(19) Europäisches Patentamt
European Patent Office
Office européen des brevets

(11) Veröffentlichungsnummer: **0 573 882 A1**

# (12) EUROPÄISCHE PATENTANMELDUNG

(21) Anmeldenummer: **93108785.2**

(22) Anmeldetag: **01.06.93**

(51) Int. Cl.[5]: **C07D 263/58**, A01N 43/76, C07D 413/12

(30) Priorität: **12.06.92 DE 4219246**

(43) Veröffentlichungstag der Anmeldung: **15.12.93 Patentblatt 93/50**

(84) Benannte Vertragsstaaten: **BE CH DE FR GB IT LI NL**

(71) Anmelder: **BAYER AG**
**D-51368 Leverkusen(DE)**

(72) Erfinder: **Förster, Heinz, Dr.**
**Am Eckbusch 47**
**W-5600 Wuppertal 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **2 -(7-Chlor-2-benzoxazolyloxy)-acetamide und ihre Verwendung als Herbizide.**

(57) Die Erfindung betrifft neue 2-(7-Chlor-2-benzoxazolyloxy)-acetamide der Formel (I),

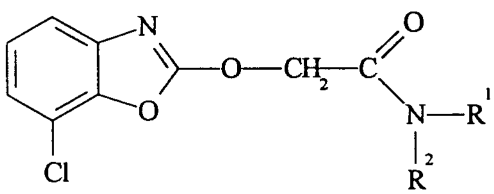

(I)

in welcher

| | |
|---|---|
| $R^1$ | für Wasserstoff oder für jeweils gegebenenfalls substituiertes Alkyl, Alkenyl oder Alkinyl steht und |
| $R^2$ | für jeweils gegebenenfalls substituiertes Alkyl, Alkenyl, Alkinyl, Alkoxy, Alkenyloxy, Alkinyloxy, Cycloalkyl, Cycloalkyloxy, Cycloalkenyl oder Aryl steht oder |
| $R^1$ und $R^2$ | gemeinsam mit dem Stickstoffatom, an welches sie gebunden sind, für einen gegebenenfalls substituierten und/oder benzoannellierten, gesättigten oder ungesättigten Heterocyclus stehen, der gegebenenfalls weitere Heteroatome enthalten kann, |

(wobei jedoch die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind),
ein Verfahren zu ihrer Herstellung und ihre Verwendung als Herbizide.

EP 0 573 882 A1

Die Erfindung betrifft neue 2-(7-Chlor-2-benzoxazolyloxy)-acetamide, ein Verfahren zu ihrer Herstellung und ihre Verwendung als Herbizide.

Es ist bekannt, daß bestimmte Heteroaryloxyacetamide wie beispielsweise die Verbindung 2-(2-Benzthiazolyloxy)-N-methyl-N-phenyl-acetamid herbizide Eigenschaften besitzen (vergl. z.B. DE-A-28 22 155; EP-A-5501; US-A-4.509.971 und US-A-4.833.243).

Bekannt sind auch 2-(5-Chlor-2-benzoxazolyloxy)-acetamide (vergl. z.B. DE-A-29 46 524; EP-A-29 171; US-A-4.408.055; DE-A-30 38 652; DE-Patentanmeldung P 41 33 673.9 vom 11.10.1991) und 2-(6-Chlor-2-benzoxazolyloxy)-acetamide (vergl. z.B DE-A-34 18 168; EP-A-161 602; US-A-4.784.682; DE-A-37 24 467; DE-Patentanmeldung P 41 33 673.9 vom 11.10.1991), ferner im Benzolring gegebenenfalls halogenierte (2-Benzoxazolyloxy)-N-benzyloxy-acetamide (vergl. z.B. JP-Anmeldung Hei 3-204,867 und WO 91/06544) sowie deren herbizide Eigenschaften. 2-(7-Chlor-2-benzthiazolyloxy)-acetamide und ihre Verwendung als Herbizide sind Gegenstand einer älteren, jedoch nicht vorveröffentlichten DE-Patentanmeldung (P 41 13 421.4 vom 25.04.1991).

Die herbizide Wirksamkeit dieser vorbekannten Verbindungen gegenüber Problemunkräutem ist jedoch ebenso wie ihre Verträglichkeit gegenüber wichtigen Kulturpflanzen nicht in allen Anwendungsgebieten völlig zufriedenstellend.

Es wurden neue 2-(7-Chlor-2-benzoxazolyloxy)-acetamide der allgemeinen Formel (I) gefunden,

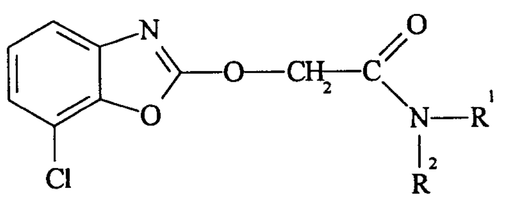

(I)

in welcher

| | |
|---|---|
| $R^1$ | für Wasserstoff oder für jeweils gegebenenfalls substituiertes Alkyl, Alkenyl oder Alkinyl steht und |
| $R^2$ | für jeweils gegebenenfalls substituiertes Alkyl, Alkenyl, Alkinyl, Alkoxy, Alkenyloxy, Alkinyloxy, Cycloalkyl, Cycloalkyloxy, Cycloalkenyl oder Aryl steht oder |
| $R^1$ und $R^2$ | gemeinsam mit dem Stickstoffatom, an welches sie gebunden sind, für einen gegebenenfalls substituierten und/oder benzoannellierten, gesättigten oder ungesättigten Heterocyclus stehen, der gegebenenfalls weitere Heteroatome enthalten kann, |

wobei jedoch die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid (vergl. z.B. JP Hei 3-204,867) ausgenommen sind.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 2-(7-Chlor-2-benzoxazolyloxy)-acetamide der allgemeinen Formel (I),

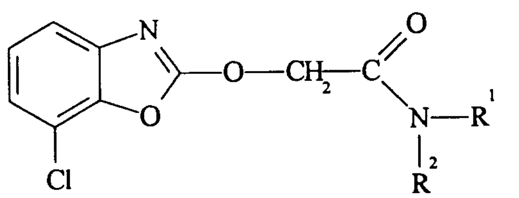

(I)

in welcher

| | |
|---|---|
| $R^1$ | für Wasserstoff oder für jeweils gegebenenfalls substituiertes Alkyl, Alkenyl oder Alkinyl steht und |

$R^2$ für jeweils gegebenenfalls substituiertes Alkyl, Alkenyl, Alkinyl, Alkoxy, Alkenyloxy, Alkinyloxy, Cycloalkyl, Cycloalkyloxy, Cycloalkenyl oder Aryl steht oder

$R^1$ und $R^2$ gemeinsam mit dem Stickstoffatom, an welches sie gebunden sind, für einen gegebenenfalls substituierten und/oder benzoannellierten, gesättigten oder ungesättigten Heterocyclus stehen, der gegebenenfalls weitere Heteroatome enthalten kann,

wobei jedoch die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind,
erhält, wenn man 2,7-Dichlorbenzoxazol der Formel (II),

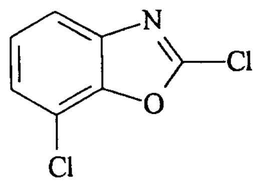

(II)

mit Hydroxyacetamiden der Formel (III),

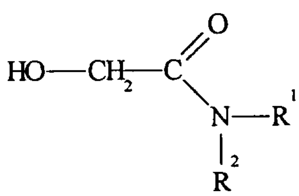

(III)

in welcher

$R^1$ und $R^2$ die oben angegebene Bedeutung haben,

gegebenenfalls in Gegenwart eines Verdünnungsmittels, gegebenenfalls in Gegenwart eines Reaktionshilfsmittels und gegebenenfalls in Gegenwart eines Katalysators umsetzt.

Schließlich wurde gefunden, daß die neuen 2-(7-Chlor-2-benzoxazolyloxy)-acetamide der allgemeinen Formel (I) herbizide Eigenschaften besitzen.

Überraschenderweise zeigen die erfindungsgemäßen Verbindungen der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Heteroaryloxyacetamiden, wie beispielsweise die Verbindung 2-(2-Benzthiazolyloxy)-N-methyl-N-phenyl-acetamid, welche chemisch und wirkungsmäßig naheliegende Verbindungen sind.

Die erfindungsgemäßen 2-(7-Chlor-2-benzoxazolyloxy)-acetamide sind durch die Formel (I) allgemein definiert. Bevorzugt sind Verbindungen der Formel (I), bei welchen

$R^1$ für Wasserstoff, für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen, Cyano, geradkettiges oder verzweigtes Alkoxy mit 1 bis 6 Kohlenstoffatomen oder Phenyl;
außerdem für gegebenenfalls einfach oder mehrfach, gleich oder verschieden durch Halogen substituiertes, geradkettiges oder verzweigtes Alkenyl mit 2 bis 8 Kohlenstoffatomen steht und
außerdem für gegebenenfalls einfach oder mehrfach, gleich oder verschieden durch Halogen substituiertes, geradkettiges oder verzweigtes Alkinyl mit 2 bis 8 Kohlenstoffatomen steht und

$R^2$ für gegebenenfalls einfach oder mehrfach, gleich oder verschieden substituiertes, geradkettiges oder verzweigtes Alkyl mit 1 bis 8 Kohlenstoffatomen
steht, wobei als Substituenten infrage kommen:
Halogen, Cyano, geradkettiges oder verzweigtes Alkoxy mit 1 bis 6 Kohlenstoffatomen,
außerdem für gegebenenfalls einfach oder mehrfach, gleich oder verschieden durch Halogen substituiertes, geradkettiges oder verzweigtes Alkenyl mit 2 bis 8 Kohlenstoffatomen steht,
außerdem für gegebenenfalls einfach oder mehrfach, gleich oder verschieden durch Halogen substituiertes, geradkettiges oder verzweigtes Alkinyl mit 2 bis 8 Kohlenstoffatomen steht,
außerdem für gegebenenfalls, einfach oder mehrfach, gleich oder verschieden substituiertes Cycloalkyl oder Cycloalkyloxy mit jeweils 3 bis 7 Kohlenstoffatomen steht, wobei als Substituenten infrage kommen:
Halogen oder geradkettiges oder verzweigtes Alkyl mit 1 bis 6 Kohlenstoffatomen,
außerdem für Cycloalkenyl mit 3 bis 7 Kohlenstoffatomen steht,
außerdem für gegebenenfalls einfach oder mehrfach, gleich oder verschieden durch geradkettiges oder verzweigtes Alkoxy mit 1 bis 6 Kohlenstoffatomen substituiertes, geradkettiges oder verzweigtes Alkoxy mit 1 bis 8 Kohlenstoffatomen steht,
außerdem für geradkettiges oder verzweigtes Alkenyloxy mit 2 bis 6 Kohlenstoffatomen steht und
außerdem für jeweils im Arylteil gegebenenfalls einfach oder mehrfach, gleich oder verschieden substituiertes Aralkyl, Aralkyloxy, Aryl oder Aryloxy mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder
Alkoximinoalkyl mit jeweils 1 bis 4 Kohlenstoffatomen in den einzelnen Alkyl teilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
oder

$R^1$ und $R^2$ gemeinsam mit dem Stickstoffatom, an welches sie gebunden sind, für einen gegebenenfalls einfach oder mehrfach, gleich oder verschieden substituierten, gesättigten oder ungesättigten und/oder benzoannellierten Heterocyclus stehen, der gegebenenfalls 1 bis 3 weitere Heteroatome - insbesondere Stickstoff, Sauerstoff und/oder Schwefel - enthalten kann, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
wobei jedoch die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

Besonders bevorzugt sind Verbindungen der Formel (I), bei welchen

$R^1$ für Wasserstoff, für gegebenenfalls einfach bis dreifach, gleich oder verschieden substituiertes, geradkettiges oder verzweigtes Alkyl mit 1 bis 6 Kohlenstoffatomen steht, wobei als Substituenten infrage kommen:
Halogen, Cyano, geradkettiges oder verzweigtes Alkoxy mit 1 bis 4 Kohlenstoffatomen oder Phenyl;
außerdem für gegebenenfalls einfach bis dreifach, gleich oder verschieden durch Halogen substituiertes, geradkettiges oder verzweigtes Alkenyl mit 2 bis 6 Kohlenstoffatomen steht und
außerdem für gegebenenfalls einfach bis dreifach, gleich oder verschieden durch Halogen substituiertes, geradkettiges oder verzweigtes Alkinyl mit 2 bis 6 Kohlenstoffatomen steht und

$R^2$ für gegebenenfalls einfach bis dreifach, gleich oder verschieden substituiertes, geradkettiges oder verzweigtes Alkyl mit 1 bis 6 Kohlenstoffatomen steht, wobei als Substituenten infrage kommen:

Halogen, Cyano, geradkettiges oder verzweigtes Alkoxy mit 1 bis 6 Kohlenstoffatomen, außerdem für gegebenenfalls einfach bis dreifach, gleich oder verschieden durch Halogen substituiertes, geradkettiges oder verzweigtes Alkenyl mit 2 bis 6 Kohlenstoffatomen steht, außerdem für gegebenenfalls einfach bis dreifach, gleich oder verschieden durch Halogen substituiertes, geradkettiges oder verzweigtes Alkinyl mit 2 bis 6 Kohlenstoffatomen steht, außerdem für gegebenenfalls, einfach bis dreifach, gleich oder verschieden substituiertes Cycloalkyl oder Cycloalkyloxy mit jeweils 3 bis 6 Kohlenstoffatomen steht, wobei als Substituenten infrage kommen:
Halogen oder geradkettiges oder verzweigtes Alkyl mit 1 bis 4 Kohlenstoffatomen, außerdem für Cycloalkenyl mit 3 bis 6 Kohlenstoffatomen steht, außerdem für gegebenenfalls einfach oder zweifach, gleich oder verschieden durch geradkettiges oder verzweigtes Alkoxy mit 1 bis 4 Kohlenstoffatomen substituiertes, geradkettiges oder verzweigtes Alkoxy mit 1 bis 6 Kohlenstoffatomen steht, außerdem für geradkettiges oder verzweigtes Alkenyloxy mit 2 bis 6 Kohlenstoffatomen steht und außerdem für jeweils im Phenylteil gegebenenfalls einfach bis dreifach, gleich oder verschieden substituiertes Phenylalkyl, Phenylalkyloxy, Phenyl oder Phenoxy mit gegebenenfalls 1 bis 4 Kohlenstoffatomen im geradkettigen oder verzweigten Alkylteil steht, wobei als Phenylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; oder

$R^1$ und $R^2$ gemeinsam mit dem Stickstoffatom, an welches sie gebunden sind, für einen gegebenenfalls einfach bis dreifach, gleich oder verschieden substituierten, gesättigten oder ungesättigten und/oder benzoannellierten Heterocyclus stehen, der gegebenenfalls 1 oder 2 weitere Heteroatome - insbesondere Stickstoff, Sauerstoff und/oder Schwefel - enthalten kann, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
wobei jedoch die Verbindungen 2-(7-Chlor-2-benzoxazolyloxy)-N-ethyl-N-benzyl-oxy-acetamid und 2-(7-Chlor-2-benzoxazolyloxy)-N-(2-n-butoxyethyl)-N-benzyloxy-acetamid ausgenommen sind.

Ganz besonders bevorzugt sind Verbindungen der Formel (I), bei welchen

$R^1$ für geradkettiges oder verzweigtes Alkyl mit 1 bis 4 Kohlenstoffatomen, für Trifluorethyl, Trifluorpropyl, Cyanethyl, Allyl Propargyl, Methoxymethyl, Methoryethyl, Methoxypropyl, Ethoxymethyl, Ethoxyethyl, Ethoxypropyl oder Benzyl steht und

$R^2$ für gegebenenfalls einfach bis dreifach, gleich oder verschieden durch Fluor und/oder Chlor substituiertes, geradkettiges oder verzweigtes Alkyl mit 1 bis 6 Kohlenstoffatomen steht, außerdem für Cyanmethyl, Cyanethyl oder Cyanpropyl steht, außerdem für geradkettiges oder verzweigtes Alkoxyalkyl mit jeweils 1 bis 3 Kohlenstoffatomen in den einzelnen Alkylteilen, für Allyl, Propargyl, Cyclopentyl, Cyclohexyl, Cyclohexyloxy, Cyclohexenyl, für geradkettiges oder verzweigtes Alkoxy mit 1 bis 6 Kohlenstoffatomen oder für geradkettiges oder verzweigtes Alkoxyalkoxy mit jeweils 1 bis 3 Kohlenstoffatomen in den einzelnen Alkylteilen, für Allyloxy, Propargyloxy oder für jeweils im Phenylteil gegebenenfalls einfach oder zweifach, gleich oder verschieden substituiertes Benzyl, Phenylethyl, Benzyloxy, Phenylethyloxy, Phenyl oder Phenoxy steht, wobei als Phenylsubstituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio; oder

$R^1$ und $R^2$ gemeinsam mit dem Stickstoffatom, an welches sie gebunden sind, für einen jeweils gegebenenfalls einfach bis dreifach, gleich oder verschieden durch Methyl und/oder Ethyl substituierten 1-Piperidinyl-, 1-Pyrrolidinyl-, 1-Perhydroazepinyl-, 4-Morpholinyl-, 1-(1,2,3,4-Tetrahydrochinolyl)- oder 2-(1,2,3,4-Tetrahydroisochinolyl)-rest stehen,
wobei jedoch die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

Im einzelnen seien außer den bei den Herstellungsbeispielen genannten Verbindungen die folgenden 2-(7-Chlor-2-benzoxazolyloxy)-acetamide der allgemeinen Formel (I) genannt:

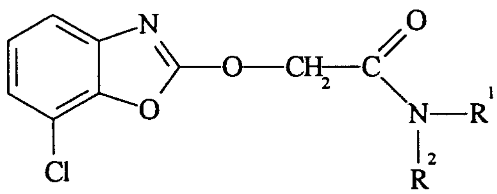

(I)

Tabelle 1:

| $R^1$ | $R^2$ | $R^1$ | $R^2$ |
|---|---|---|---|
| $CH_3$ | $C_2H_5$ | $CH_3$ | 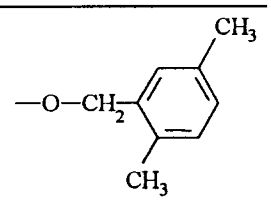 |
| i-$C_3H_7$ | $C_2H_5$ | $CH_3$ | 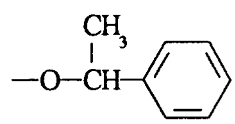 |
| $C_2H_5$ | -O-n-$C_3H_7$ | n-$C_3H_7$ | -O-n-$C_4H_9$ |
| $C_2H_5$ | -O-n-$C_4H_9$ | n-$C_3H_7$ | -O-i-$C_4H_9$ |
| $C_2H_5$ | -O-i-$C_3H_7$ | n-$C_3H_7$ | -O-s-$C_4H_9$ |
| $C_2H_5$ | -O-i-$C_4H_9$ | $C_2H_5$ | -O-s-$C_4H_9$ |

Tabelle 1 (Fortsetzung):

| $R^1$ | $R^2$ | $R^1$ | $R^2$ |
|---|---|---|---|
| i-$C_3H_7$ | n-$C_3H_7$ | $CH_3$ | 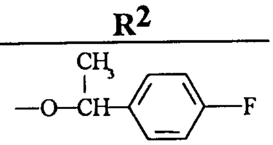 |
| $CH_3$ | 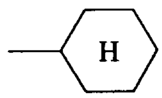 | i-$C_3H_7$ | 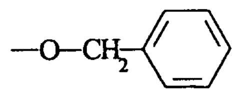 |
| i-$C_3H_7$ | 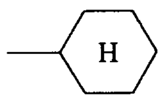 | i-$C_3H_7$ | 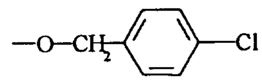 |
| $CH_3$ | -$CH_2$-$C_6H_5$ | i-$C_3H_7$ | 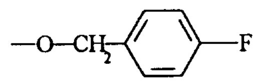 |
| $CH_3$ | 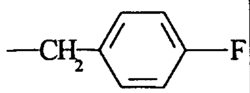 | i-$C_3H_7$ | 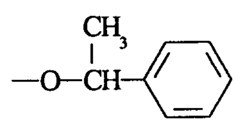 |
| $CH_3$ | 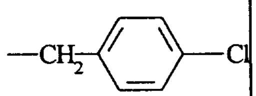 | i-$C_3H_7$ | 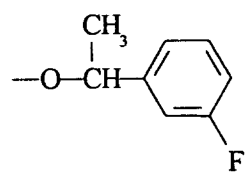 |
| $CH_3$ | 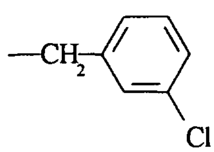 | i-$C_3H_7$ | 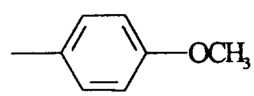 |
| $CH_3$ | 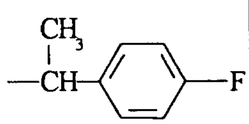 | i-$C_3H_7$ | 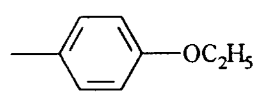 |
| $CH_3$ | 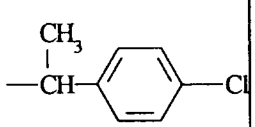 | $CH_3$ | 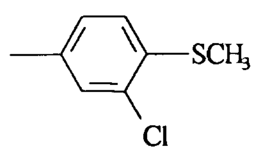 |

Tabelle 1 (Fortsetzung):

| $R^1$ | $R^2$ | $R^1$ | $R^2$ |
|---|---|---|---|
| $CH_3$ | 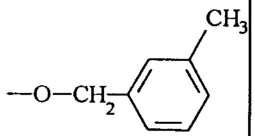 | $i\text{-}C_3H_7$ | 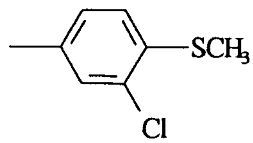 |
| $i\text{-}C_3H_7$ | 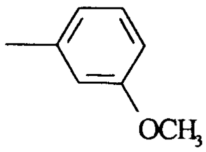 | $i\text{-}C_3H_7$ | 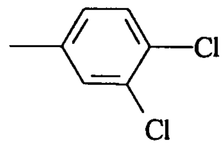 |
| $i\text{-}C_3H_7$ | 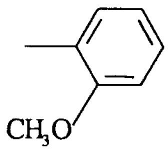 | $i\text{-}C_3H_7$ | 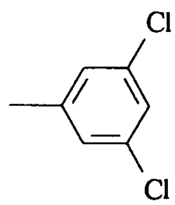 |
| $CH_3$ | 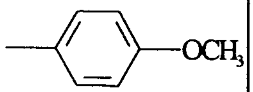 | $i\text{-}C_3H_7$ | 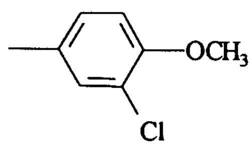 |
| $CH_3$ | 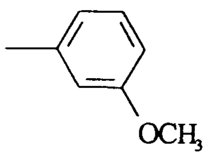 | $i\text{-}C_3H_7$ | 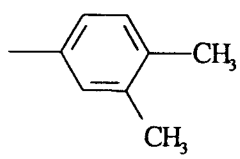 |
| $CH_3$ | 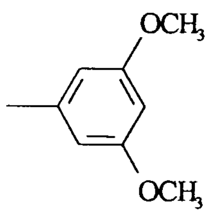 | $CH_3$ | $-CH_2-CH_2-CN$ |
| $CH_3$ | 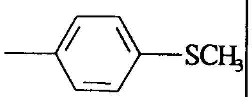 | $-CH_2-CH_2-CN$ | $-CH_2-CH_2-CN$ |

Tabelle 1 (Fortsetzung):

| $R^1$ | $R^2$ | $R^1$ | $R^2$ |
|---|---|---|---|
| $i-C_3H_7$ | 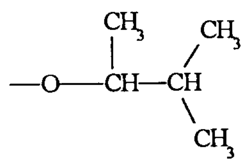 | $i-C_3H_7$ | $-CH_2-CH_2-OCH_3$ |
| $i-C_3H_7$ | $-O-n-C_6H_{13}$ | $i-C_3H_7$ | $-O-n-C_4H_9$ |
| $i-C_3H_7$ | 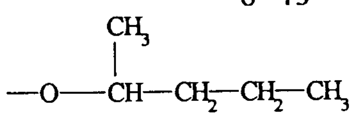 | $i-C_3H_7$ | $-O-i-C_4H_9$ |
| $i-C_3H_7$ | $-O-(CH_2)_3-CH(CH_3)_2$ | $i-C_3H_7$ | $-O-s-C_4H_9$ |
| $i-C_3H_7$ | $-O-(CH_2)_2-CH(CH_3)_2$ | $i-C_3H_7$ | $-O-n-C_5H_{11}$ |
| $i-C_3H_7$ | 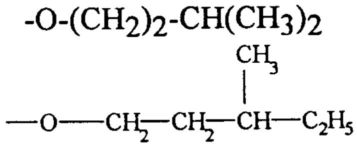 | $CH_3$ | $-O-CH_2-CH_2-OCH_3$ |
| $i-C_3H_7$ | 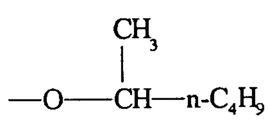 | 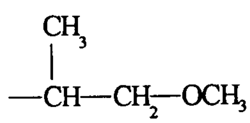 | $C_6H_5$ |
| $i-C_3H_7$ | 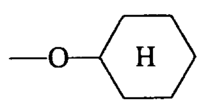 | 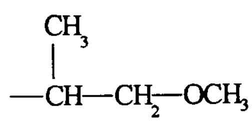 | 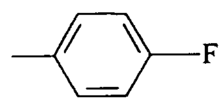 |
| $n-C_3H_7$ | $-O-CH_2-CH_2-OCH_3$ | $CH_3$ | $-O-C_2H_5$ |
| $i-C_3H_7$ | $-O-CH_2-CH_2-O-i-C_3H_7$ | $CH_3$ | $-O-n-C_3H_7$ |
| $n-C_3H_7$ | $-O-CH_2-CH_2-O-C_2H_5$ | $CH_3$ | $-O-n-C_4H_9$ |
| $C_2H_5$ | $-O-C_2H_5$ | $n-C_3H_7$ | $-O-n-C_3H_7$ |

Verwendet man beispielsweise 2,7-Dichlorbenzoxazol und 2-Hydroxyessigsäure-N-methylanilid als Ausgangsstoffe, so läßt sich der Reaktionsablauf des erfindungsgemäßen Verfahrens durch das folgende Formelschema darstellen:

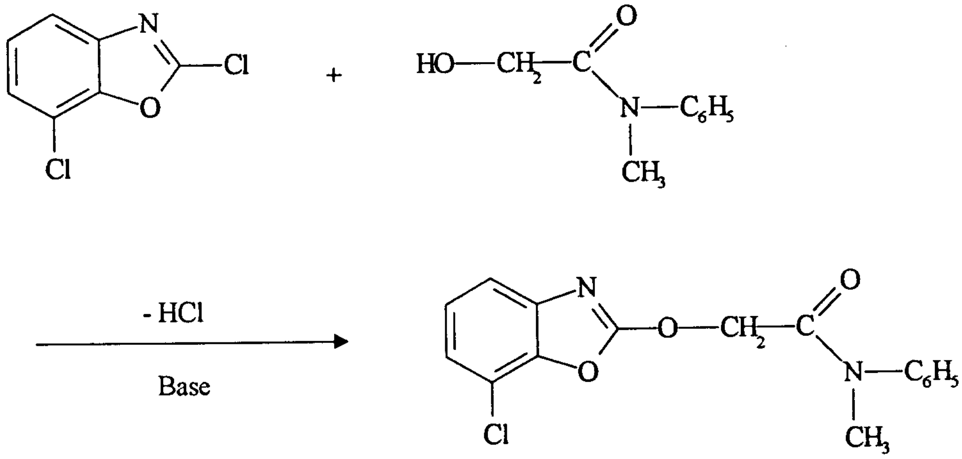


Die zur Durchführung des erfindungsgemäßen Verfahrens als Ausgangsverbindung benötigte 2,7-Dichlorbenzoxazol ist durch die Formel (II) definiert. 2,7-Dichlorbenzoxazol der Formel (II) ist bekannt (vergl. z.B. US 4.476.137).

Die zur Durchführung des erfindungsgemäßen Verfahrens weiterhin als Ausgangsprodukte erforderlichen Hydroxyacetamide sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.
Die Hydroxyacetamide der Formel (III) sind bekannt oder erhältlich in Analogie zu bekannten Verfahren (vergl. z.B. DE 41 13 421; US 4.509.971; US 4.645.525; US 4.334.073; DE 30 38 598; DE 30 38 636; EP 37 526; EP 348 737; DE 38 19 477).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chiorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole; wie Methanol, Ethanol, n- oder i-Propanol, deren Gemische mit Wasser, reines Wasser oder wässrige Salzlösungen. Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt sind wässrige Natriumchloridlösungen.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}/C_{15}$-alkylammoniumchlorid, Trimethyl-$C_{13}/C_{15}$-alkylammoniumbromid, Dibenzyl-dimethyl-ammoniummethylsulfat, Dimethyl-$C_{12}/C_{14}$-alkyl-benzylammoniumchlorid, Dimethyl-$C_{12}/C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des erfindungsgemäßen Verfahrens in einem größeren Bereich variiert werden. Im allgemeinen arbeitet man bei Temperaturen zwischen -50°C und 110°C, vorzugsweise bei Temperaturen zwischen -20°C und +80°C.

Zur Durchführung des erfindungsgemäßen Verfahrens setzt man pro Mol 2,7-Dichlorbenzoxazol der Formel (II) im allgemeinen 0,5 bis 5,0 Mol, vorzugsweise 0,8 bis 1,5 Mol Hydroxyacetamid der Formel (III), 0,5 bis 5,0 Mol, vorzugsweise 0,8 bis 1,5 Mol als Reaktionshilfsmittel verwendeter Base und gegebenenfalls 0,001 bis 2,0 Mol, vorzugsweise 0,001 bis 1,0 Mol Phasentransferkatalysator ein. Die Reaktionsdurchführung, Aufarbeitung und Isolierung der Reaktionsprodukte erfolgt nach bekannten Verfahren (vergl. hierzu beispielsweise DE 41 13 421 oder die Herstellungsbeispiele).

Die Reinigung der Endprodukte der Formel (I) erfolgt mit Hilfe üblicher Verfahren, beispielsweise durch Säulenchromatographie oder durch Umkristallisieren. Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindemia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. aufIndustrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weidenflächen und zur selektiven Unkrautbekämpfung in einjährigen Kulturen eingesetzt werden.

Dabei lassen sich die erfindungsgemäßen Wirkstoffe mit besonders gutem-Erfolg zur Bekämpfung von monokotylen Unkräutern monokotylen Kulturen wie beispielsweise Reis einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith,

Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.
Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.
Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochioridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beipiel 1:

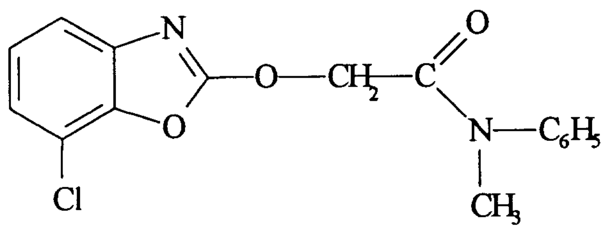

(Verfahren a)

Zu einer Lösung von 3,3 g (0,02 Mol) Hydroxyessigsäure-N-methylanilid und 2,2 g (0,02 Mol) Kalium-t-butylat in 40 ml t-Butanol und 15 ml Acetonitril gibt man bei 0 °C bis 5 °C tropfenweise unter Rühren eine Lösung aus 3,8 g (0,02 Mol) 2,7-Dichlor-benzoxazol in 5 ml Acetonitril und rührt nach beendeter Zugabe weitere 10 Stunden bei 0°C bis 5 °C. Zur Aufarbeitung gibt man Wasser zur Reaktionsmischung, saugt ausgefallenen Niederschlag ab und trocknet ihn.

Man erhält 4,6 g (72 % der Theorie) 2-[(7-Chlor-2-benzoxazolyl)-oxy]-N-methylacetanilid vom Schmelzpunkt 143°C.

In entsprechender Weise erhält man die folgenden 2-(7-Chlor-2-benzoxazolyloxy)-acetamide der allgemeinen Formel (I):

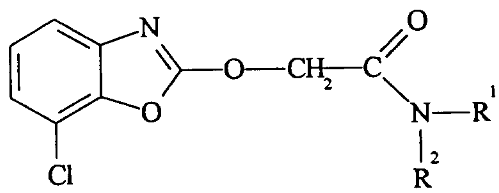

(I)

Tabelle 2:

| Beispiel-Nr. | $R^1$ | $R^2$ | physikalische Eigenschaften |
|---|---|---|---|
| 2 | i-$C_3H_7$ | $C_6H_5$ | Fp. 129°C |
| 3 | i-$C_3H_7$ | 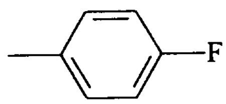 | Fp. 122°C |
| 4 | $CH_3$ | 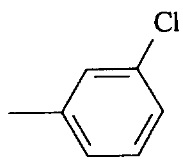 | Fp. 122°C |
| 5 | $CH_3$ | 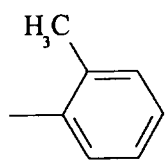 | Fp. 132°C |
| 6 | $CH_3$ | 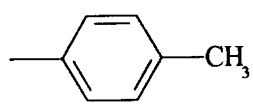 | Fp. 75°C |
| 7 | 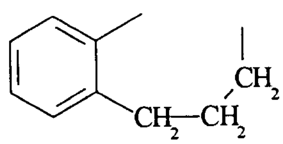 | | Fp. 77°C |

Tabelle 2 (Fortsetzung):

| Beispiel-Nr. | $R^1$ | $R^2$ | physikalische Eigenschaften |
|---|---|---|---|
| 8 | $n\text{-}C_3H_7$ | $C_6H_5$ | Fp. 138°C |
| 9 | $CH_3$ | 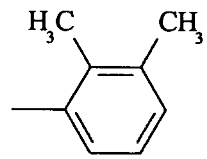 | Fp. 145°C |
| 10 | 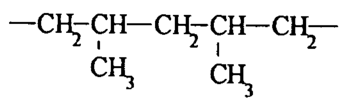 | | $n_D^{20}$ = 1,5408 |
| 11 | $-CH_2-CH_2-CH_2-CH_2-CH_2-CH_2-$ | | Fp. 58°C |
| 12 | $C_2H_5$ | $C_6H_5$ | Fp. 88°C |
| 13 | $n\text{-}C_3H_7$ | $n\text{-}C_3H_7$ | Fp. 70°C |
| 14 | $-CH_2-CH=CH_2$ | $-CH_2-CH=CH_2$ | Fp. 50°C |
| 15 | $CH_3$ | $n\text{-}C_4H_9$ | Fp. 43°C |
| 16 | $CH_3$ | 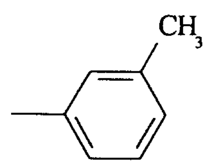 | Fp. 112°C |
| 17 | $n\text{-}C_4H_9$ | $n\text{-}C_4H_9$ | Fp. 101°C |
| 18 | $CH_3$ | 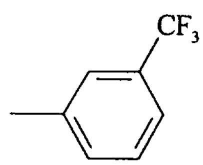 | Fp. 103°C |
| 19 | $CH_3$ | 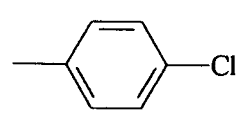 | Fp. 101°C |

Tabelle 2 (Fortsetzung):

| Beispiel-Nr. | $R^1$ | $R^2$ | physikalische Eigenschaften |
|---|---|---|---|
| 20 | $CH_3$ | $s\text{-}C_4H_9$ | $n_D^{20} = 1{,}5308$ |
| 21 | $i\text{-}C_3H_7$ | 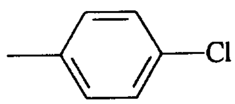 | Fp. 82°C |
| 22 | $CH_3$ | $-CH_2\text{-}CF_3$ | Fp. 70°C |
| 23 | 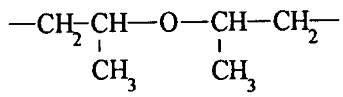 | | Fp. 75°C |

Anwendungsbeispiele:

In den folgenden Anwendungsbeispielen wurde die nachstehend aufgeführte Verbindung als Vergleichssubstanz eingesetzt:

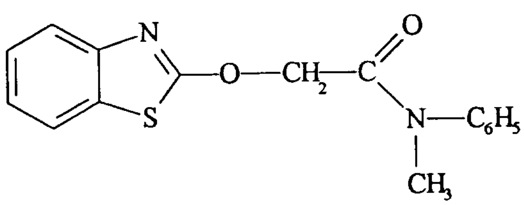

(A)

2-(2-Benzthiazolyloxy)-N-methyl-N-phenyl-acetamid (bekannt aus DE 28 22 155)

Beispiel A:

**Pre-emergence-Wasseroberflächenbehandlung bei verpflanztem Wasserreis**

Lösungsmittel: 5 Gewichtsteile Aceton
Emulgator: 1 Gewichtsteil Alkylarylpolyglykolether

Zur Herstellung einer applizierfähigen Wirkstoffzubereitung wird ein Gewichtsteil Wirkstoff in der angegebenen Menge Lösungsmittel gelöst, anschließend die angegebene Menge Emulgator zugegeben und das Konzentrat mit Wasser auf die gewünschte Konzentration verdünnt.

In Töpfe, die mit Erde gefüllt sind, wird Reis im 2-3-Blattstadium verpflanzt. Samen von Testpflanzen werden ausgelegt (1 cm tief). Zwei Tage später werden die Töpfe mit Wasser 3 cm hoch überstaut. Anschließend wird die Wirkstoffzubereitung auf die Wasseroberfläche ausgebracht. 4 Wochen später wird die herbizide Wirkung und die Schädigung der behandelten Pflanzen im Vergleich zur unbehandelten Kontrolle visuell ausgewertet.

0 % = keine Wirkung (wie unbehandelte Kontrolle)
100 % = totale Vernichtung

Eine deutliche Überlegenheit in der Wirksamkeit gegenüber Schadpflanzen wie beispielsweise Hühnerhirse (Echinochloa crus galli) bei vergleichbarer Verträglichkeit gegenüber Reis im Vergleich zu den aus dem Stand der Technik bekannten Verbindungen zeigen in diesem Test z.B. die Verbindungen gemäß den Herstellungsbeispielen 1, 2, 6, 7, 8, 9, 12, 17, 18, 19 und 21.

**Patentansprüche**

1. 2-(7-Chlor-2-benzoxazolyloxy)-acetamide der allgemeinen Formel (I),

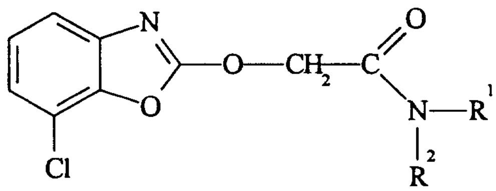

(I)

in welcher

$R^1$ für Wasserstoff oder für jeweils gegebenenfalls substituiertes Alkyl, Alkenyl oder Alkinyl steht und

$R^2$ für jeweils gegebenenfalls substituiertes Alkyl, Alkenyl, Alkinyl, Alkoxy, Alkenyloxy, Alkinyloxy, Cycloalkyl, Cycloalkyloxy, Cycloalkenyl oder Aryl steht oder

$R^1$ und $R^2$ gemeinsam mit dem Stickstoffatom, an welches sie gebunden sind, für einen gegebenenfalls substituierten und/oder benzoannellierten, gesättigten oder ungesättigten Heterocyclus stehen, der gegebenenfalls weitere Heteroatome enthalten kann,

wobei die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

2. Verbindungen der Formel (I) gemäß Anspruch 1, dadurch gekennzeichnet, daß in Formel (I)

$R^1$ für Wasserstoff, für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen: Halogen, Cyano, geradkettiges oder verzweigtes Alkoxy mit 1 bis 6 Kohlenstoffatomen oder Phenyl;
außerdem für gegebenenfalls einfach oder mehrfach, gleich oder verschieden durch Halogen substituiertes, geradkettiges oder verzweigtes Alkenyl mit 2 bis 8 Kohlenstoffatomen steht und
außerdem für gegebenenfalls einfach oder mehrfach, gleich oder verschieden durch Halogen substituiertes, geradkettiges oder verzweigtes Alkinyl mit 2 bis 8 Kohlenstoffatomen steht und

$R^2$ für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen, Cyano, geradkettiges oder verzweigtes Alkoxy mit 1 bis 6 Kohlenstoffatomen,
außerdem für gegebenenfalls einfach oder mehrfach, gleich oder verschieden durch Halogen substituiertes, geradkettiges oder verzweigtes Alkenyl mit 2 bis 8 Kohlenstoffatomen steht,
außerdem für gegebenenfalls einfach oder mehrfach, gleich oder verschieden durch Halogen substituiertes, geradkettiges oder verzweigtes Alkinyl mit 2 bis 8 Kohlenstoffatomen steht,
außerdem für gegebenenfalls, einfach oder mehrfach, gleich oder verschieden substituiertes Cycloalkyl oder Cycloalkyloxy mit jeweils 3 bis 7 Kohlenstoffatomen steht, wobei als Substituenten infrage kommen:
Halogen oder geradkettiges oder verzweigtes Alkyl mit 1 bis 6 Kohlenstoffatomen,
außerdem für Cycloalkenyl mit 3 bis 7 Kohlenstoffatomen steht,
außerdem für gegebenenfalls einfach oder mehrfach, gleich oder verschieden durch geradkettiges oder verzweigtes Alkoxy mit 1 bis 6 Kohlenstoffatomen substituiertes, geradkettiges oder verzweigtes Alkoxy mit 1 bis 8 Kohlenstoffatomen steht,
außerdem für geradkettiges oder verzweigtes Alkenyloxy mit 2 bis 6 Kohlenstoffatomen steht und
außerdem für jeweils im Arylteil gegebenenfalls einfach oder mehrfach, gleich oder

verschieden substituiertes Aralkyl, Aralkyloxy, Aryl oder Aryloxy mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkyl-thio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; oder

$R^1$ und $R^2$ gemeinsam mit dem Stickstoffatom, an welches sie gebunden sind, für einen gegebenenfalls einfach oder mehrfach, gleich oder verschieden substituierten, gesättigten oder ungesättigten und/oder benzoannellierten Heterocyclus stehen, der gegebenenfalls 1 bis 3 weitere Heteroatome - insbesondere Stickstoff, Sauerstoff und/oder Schwefel - enthalten kann, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkyl-thio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,
wobei die Verbindungen 2⁻(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

**3.** Verbindungen der Formel (I) gemäß Anspruch 1, dadurch gekennzeichnet, daß in Formel (I)

$R^1$ für Wasserstoff, für gegebenenfalls einfach bis dreifach, gleich oder verschieden substituiertes, geradkettiges oder verzweigtes Alkyl mit 1 bis 6 Kohlenstoffatomen steht, wobei als Substituenten infrage kommen:
Halogen, Cyano, geradkettiges oder verzweigtes Alkoxy mit 1 bis 4 Kohlenstoffatomen oder Phenyl;
außerdem für gegebenenfalls einfach bis dreifach, gleich oder verschieden durch Halogen substituiertes, geradkettiges oder verzweigtes Alkenyl mit 2 bis 6 Kohlenstoffatomen steht und
außerdem für gegebenenfalls einfach bis dreifach, gleich oder verschieden durch Halogen substituiertes, geradkettiges oder verzweigtes Alkinyl mit 2 bis 6 Kohlenstoffatomen steht und

$R^2$ für gegebenenfalls einfach bis dreifach, gleich oder verschieden substituiertes, geradkettiges oder verzweigtes Alkyl mit 1 bis 6 Kohlenstoffatomen steht, wobei als Substituenten infrage kommen:
Halogen, Cyano, geradkettiges oder verzweigtes Alkoxy mit 1 bis 6 Kohlenstoffatomen,
außerdem für gegebenenfalls einfach bis dreifach, gleich oder verschieden durch Halogen substituiertes, geradkettiges oder verzweigtes Alkenyl mit 2 bis 6 Kohlenstoffatomen steht,
außerdem für gegebenenfalls einfach bis dreifach, gleich oder verschieden durch Halogen substituiertes, geradkettiges oder verzweigtes Alkinyl mit 2 bis 6 Kohlenstoffatomen steht,
außerdem für gegebenenfalls, einfach bis dreifach, gleich oder verschieden substituiertes Cycloalkyl oder Cycloalkyloxy mit jeweils 3 bis 6 Kohlenstoffatomen steht, wobei als Substituenten infrage kommen:
Halogen oder geradkettiges oder verzweigtes Alkyl mit 1 bis 4 Kohlenstoffatomen,
außerdem für Cycloalkenyl mit 3 bis 6 Kohlenstoffatomen steht,
außerdem für gegebenenfalls einfach oder zweifach, gleich oder verschieden durch

geradkettiges oder verzweigtes Alkoxy mit 1 bis 4 Kohlenstoffatomen substituiertes, geradkettiges oder verzweigtes Alkoxy mit 1 bis 6 Kohlenstoffatomen steht, außerdem für geradkettiges oder verzweigtes Alkenyloxy mit 2 bis 6 Kohlenstoffatomen steht und außerdem für jeweils im Phenylteil gegebenenfalls einfach bis dreifach, gleich oder verschieden substituiertes Phenylalkyl, Phenylalkyloxy, Phenyl oder Phenoxy mit gegebenenfalls 1 bis 4 Kohlenstoffatomen im geradkettigen oder verzweigten Alkylteil steht, wobei als Phenylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; oder

$R^1$ und $R^2$ gemeinsam mit dem Stickstoffatom, an welches sie gebunden sind, für einen gegebenenfalls einfach bis dreifach, gleich oder verschieden substituierten, gesättigten oder ungesättigten und/oder benzoannellierten Heterocyclus stehen, der gegebenenfalls 1 oder 2 weitere Heteroatome - insbesondere Stickstoff, Sauerstoff und/oder Schwefel - enthalten kann, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
wobei die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

**4.** Verbindungen der Formel (I) gemäß Anspruch 1, dadurch gekennzeichnet, daß in Formel (I)

$R^1$ für geradkettiges oder verzweigtes Alkyl mit 1 bis 4 Kohlenstoffatomen, für Trifluorethyl, Trifluorpropyl, Cyanethyl, Allyl, Propargyl, Methoxymethyl, Methoxyethyl, Methoxypropyl, Ethoxymethyl, Ethoxyethyl, Ethoxypropyl oder Benzyl steht und

$R^2$ für gegebenenfalls einfach bis dreifach, gleich oder verschieden durch Fluor und/oder Chlor substituiertes, geradkettiges oder verzweigtes Alkyl mit 1 bis 6 Kohlenstoffatomen steht, außerdem für Cyanmethyl, Cyanethyl oder Cyanpropyl steht, außerdem für geradkettiges oder verzweigtes Alkoxyalkyl mit jeweils 1 bis 3 Kohlenstoffatomen in den einzelnen Alkylteilen, für Allyl, Propargyl, Cyclopentyl, Cyclohexyl, Cyclohexyloxy, Cyclohexenyl, für geradkettiges oder verzweigtes Alkoxy mit 1 bis 6 Kohlenstoffatomen oder für geradkettiges oder verzweigtes Alkoxyalkoxy mit jeweils 1 bis 3 Kohlenstoffatomen in den einzelnen Alkylteilen, für Allyloxy, Propargyloxy oder für jeweils im Phenylteil gegebenenfalls einfach oder zweifach, gleich oder verschieden substituiertes Benzyl, Phenylethyl, Benzyloxy, Phenylethyloxy, Phenyl oder Phenoxy steht, wobei als Phenylsubstituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio; oder

$R^1$ und $R^2$ gemeinsam mit dem Stickstoffatom, an welches sie gebunden sind, für einen jeweils gegebenenfalls einfach bis dreifach, gleich oder verschieden durch Methyl und/oder Ethyl substituierten 1-Piperidinyl-, 1-Pyrrolidinyl-, 1-Perhydroazepinyl-, 4-Morpholinyl-, 1-(1,2,3,4-Tetrahydrochinolyl)- oder 2-(1,2,3,4-Tetrahydroisochinolyl)-rest stehen,
wobei die Verbindungen 2-(7-Chlor-2-benzoxazolyloxy)-N-ethyl-N-benzyloxy-acetamid und 2-(7-Chlor-2-benzoxazolyloxy)-N-(2-n-butoxyethyl)-N-benzyloxy-acetamid ausgenommen sind.

**5.** Verfahren zur Herstellung von 2-(7-Chlor-2-benzoxazolyloxy)-acetamiden der Formel (I) gemäß Anspruch 1, dadurch gekennzeichnet, daß man
2,7-Dichlorbenzoxazol der Formel (II),

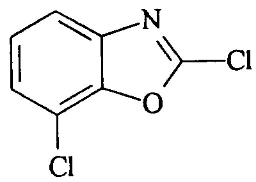

(II)

mit Hydroxyacetamiden der Formel (III),

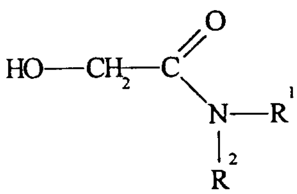

(III)

in welcher

$R^1$ und $R^2$ die oben angegebene Bedeutung haben,

gegebenenfalls in Gegenwart eines Verdünnungsmittels, gegebenenfalls in Gegenwart eines Reaktionshilfsmittels und gegebenenfalls in Gegenwart eines Katalysators umsetzt.

**6.** Herbizide Mittel, gekennzeichnet durch einen Gehalt an mindestens einer Verbindung der allgemeinen Formel (I) gemäß Anspruch 1.

**7.** Verwendung von Verbindungen der allgemeinen Formel (I) gemäß Anspruch 1 zur Bekämpfung von unerwünschtem Pflanzenwachstum.

**8.** Verfahren zur Bekämpfung von Unkräutern, dadurch gekennzeichnet, daß man Verbindungen der allgemeinen Formel (I) gemäß Anspruch 1 auf die Unkräuter und/oder ihren Lebensraum einwirken läßt.

**9.** Verfahren zur Herstellung von herbiziden Mitteln, dadurch gekennzeichnet, daß man eine Verbindung der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 93 10 8785

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | WO-A-9 106 544 (HOKKO CHEMICAL INDUSTRY CO. LTD) *Zusammenfassung;Seite7,formula1,Seite8,Verbindungen 35 und 36* | 1-9 | C07D263/58<br>A01N43/76<br>C07D413/12 |
| D,Y | EP-A-0 161 602 (BAYER AG) * Ansprüche * | 1,6-9 | |
| D,Y | DE-A-3 038 652 (BAYER AG) * Ansprüche * | 1,6-9 | |
| D,Y | EP-A-0 005 501 (BAYER AG) * Ansprüche * | 1,6-9 | |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
|---|---|---|
| DEN HAAG | 11 AUGUST 1993 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument